# EUROPEAN PATENT SPECIFICATION

(11) **EP 4 584 793 B1**
(45) Date of publication and mention of the grant of the patent: **28.10.2026**
(21) Application number: 23764641.9
(22) Date of filing: 01.09.2023
(51) Int. Cl.: G16H 20/17, G16H 40/63, A61M 5/14, A61M 5/142, A61M 5/172

(54) **SHORT-RANGE COMMUNICATING INFUSION TUBING SETS AND METHODS OF USE**
INFUSIONSSCHLAUCHSÄTZE MIT KURZSTRECKEN-KOMMUNIKATION UND VERFAHREN ZUR VERWENDUNG
ENSEMBLES DE TUBES DE PERFUSION COMMUNICANTS À COURTE PORTÉE ET PROCÉDÉS D'UTILISATION

(30) Priority: 09.09.2022 US 202263405026 P; 25.01.2023 EP 23153212
(43) Date of publication of application: 16.07.2025
(73) Proprietor: SHL Medical AG, 6300 Zug (CH)
(72) Inventor: HAWTHORNE, James Thomas, Boonton, 07005 (US)
(86) International application number: PCT/EP2023/074084
(87) International publication number: WO 2024/052241

(56) References cited:
- US-A1- 2022 223 249

## Description

### Technical Field

The present disclosure generally relates to tubing set systems, and more particularly to an improved tubing set system for infusion set tubing attachment to a medication administration system.

### Discussion

Tubing sets are integral components of parenteral administration systems. In such tubing sets, the tubing is attached to a pump which moves fluid from a reservoir through the tubing and into the patient administration site. Most commonly, these tubing sets are used to deliver intravenous (IV) or larger volume subcutaneous (SC) therapies in a clinic setting. As such, tubing sets exist in many different configurations. Tubing diameter, length, and number of branches (e.g., needle sets) may be varied to best fit patient and therapy needs.

Depending on the pump mechanism, the tubing set diameter may be used control the flow rate of the medication traveling therethrough. In these scenarios, incorrect tubing can lead to either a sub-therapeutic or supra-therapeutic administration rate. When certain medications are administered too quickly, dangerous and potentially fatal infusion-related reactions and other adverse events can occur. When administered too slowly, therapies can have a dampened therapeutic effect. This is of particular importance for time-sensitive administration such as emergency medications and pain management therapies.

A combination of the tubing diameter and length is responsible for the total volume of the tubing. The tubing diameter and length are important inputs to know when the tubing is being primed (i.e., filled with fluid to displace existing air). If too much fluid is used for priming, medication waste occurs, and the patient may not receive their full dose. If an insufficient amount of fluid is used, air remains in the tubing which can lead to a fatal air embolism if administered intravenously to the patient.

Historically, tubing sets have been managed by a healthcare professional in a healthcare setting. Despite extensive formal training, administration errors involving tubing sets are not uncommon. Medications are delivered through incorrect tubing sets, to incorrect sites, or have air in the tubing leading to complications for the patient. Fatal cases of overdoses of chemotherapeutic agents due to incorrect tubing selections are known to occur. In recent years, parenteral therapy administration has begun to shift into the home setting to reduce strain on the healthcare system and increase patient convenience. Without the extensive training that healthcare professionals receive, patients are at a higher risk of such administration errors.

Most tubing sets utilize universal luer-lock connections, allowing almost any tubing set to be connected to any administration system. While many parts of the medication administration system utilize barcode scanning or another form of secondary check to ensure proper administration, confirmation of the correct tubing sets are overlooked, as they are all intentionally interchangeable. If a patient is administering multiple medications simultaneously in a home setting, they must attach the correct tubing to the correct ports on the pump and properly prime each set, which may all have different flow rates (diameters) and priming volumes. Further complicating the problem, the tubing sets they receive from the specialty pharmacy may be of incorrect size if an error is made. To the untrained eye, identifying differences in tubing can be extremely difficult, and priming may be unintuitive to untrained users. With no method to verify that the tubing system is set up correctly, patients are at a high risk for improper administration. Improved solutions are needed to improve usability and safety: namely, to properly verify that tubing sets are the correct size, are in the correct location, are linked to the correct medication, and are primed with the correct volume.

US2022223249A1 discloses an infusion system in which an infusion container, an administration set and a pump are associated via identifiers (e.g. barcodes/RFID) and an electronic medical records (EMR) system. The system retrieves information about the content of the infusion container from the EMR, compares it with a drug identification for the planned infusion, and only permits the pump to start infusing if the content matches the prescribed drug. If a mismatch is detected, infusion is blocked and an alert is generated to reduce infusion administration line errors.

### Summary

The invention is defined by the appended claims.

The present disclosure relates to an improved system for infusion set tubing attachment to the medication administration system. More particularly, the invention relates to the facilitation and verification system to confirm one or more tubing sets are configured properly in their attachment to a medication administration system.

In particular, in a first aspect, the present disclosure provides a medication administration system comprising: (A) one or more tubes having a first end and a second end opposite the first end, the one or more tubes including: (i) at least one first communication interface configured to transmit tube information corresponding to the one or more tubes; and (B) a pump including: (i) one or more ports configured to receive the first end of the one or more tubes; (ii) at least one second communication interface configured to receive the tube information from the at least one first communication interface when the first end of the one or more tubes are positioned in the one or more ports; (iii) at least one memory storage element including a plurality of medication administration regimens associated with the medication administration system; (iv) a user interface configured to receive an indication of a given medication administration regimen of the plurality of medication administration regimens for administration to a patient; (v) at least one processor; and (vi) data storage including program instructions stored thereon that when executed by the at least one processor, cause the pump to: (a) compare the the tube information received from the one or more tubes to one or more tube parameters of the given medication administration regimen; (b) in response to a determination that the tube information received from the one or more tubes are not compatible with the one or more tube parameters of the given medication administration regimen, prevent transmission of one or more medicaments through the one or more tubes; and (c) in response to a determination that the tube information received from the one or more tubes are compatible with the one or more tube parameters of the given medication administration regimen, enable transmission of one or more medicaments through the one or more tubes.

In one embodiment of the first aspect, each of the one or more tubes includes a first communication interface.

In another embodiment of the first aspect, the first communication interface of each of the one or more tubes is positioned at the first end of each of the one or more tubes.

In another embodiment of the first aspect, the one or more tubes further comprise a housing including one or more through holes, and wherein the first end of the one or more tubes are positioned though the one or more through holes.

In one such embodiment of the first aspect, each of the one or more tubes includes a first communication interface.

In another such embodiment of the first aspect, the housing includes a single first communication interface.

In another embodiment of the first aspect, the pump is further configured to: in response to the determination that the tube information received from the one or more tubes does not match the one or more tube parameters of the given medication administration regimen, provide for display a warning message on the user interface.

In another embodiment of the first aspect, the pump is further configured to: in response to the determination that the tube information received from the one or more tubes matches the one or more tube parameters of the given medication administration regimen, automatically prime each of the one or more tubes.

In another embodiment of the first aspect, the tube information corresponding to the one or more tubes comprises one or more of a product identification parameter, a tubing flow rate, a tubing size, a tubing volume for priming, and an air or occlusion detection data.

In another embodiment of the first aspect, the one or more tube parameters of the given medication administration regimen comprises one or more of a tubing product, a tubing flow rate, a tubing size, a number of tubes, a positioning of tubes, and a tubing volume for priming.

In another embodiment of the first aspect, the tube information corresponding to the one or more tubes is provided to the at least one first communication interface during manufacturing of the one or more tubes.

In another embodiment of the first aspect, the tube information corresponding to the one or more tubes is provided to the at least one first communication interface by a healthcare professional after manufacturing of the one or more tubes.

In a second aspect, the present disclosure provides method for confirming one or more tubes of a medicament administration system, the method comprising: (a) receiving, at a user interface of a pump, an indication of a given medication administration regimen of a plurality of medication administration regimens associated with a medication administration system for administration to a patient; (b) positioning a first end of one or more tubes into one or more ports of the pump; (c) receiving, by at least one second communication interface of the pump, tube information corresponding to the one or more tubes from at least one first communication interface of the one or more tubes when the first end of the one or more tubes are positioned in the one or more ports; (d) comparing the the tube information received from the one or more tubes to one or more tube parameters of the given medication administration regimen; (e) in response to a determination that the tube information received from the one or more tubes are not compatible with the one or more tube parameters of the given medication administration regimen, preventing transmission of one or more medicaments through the one or more tubes; and (f) in response to a determination that the tube information received from the one or more tubes are compatible with the one or more tube parameters of the given medication administration regimen, enabling transmission of one or more medicaments through the one or more tubes.

In one embodiment of the second aspect, the method further includes in response to the determination that the tube information received from the one or more tubes does not match the one or more tube parameters of the given medication administration regimen, providing for display a warning message on the user interface.

In another embodiment of the second aspect, the method further includes in response to the determination that the tube information received from the one or more tubes matches the one or more tube parameters of the given medication administration regimen, automatically priming each of the one or more tubes.

These as well as other aspects, advantages, and alternatives, will become apparent to those of ordinary skill in the art by reading the following detailed description, with reference where appropriate to the accompanying drawings.

### Brief Description of the Figures

Figure 1 illustrates a simplified block diagram of a medication administration system, according to an example embodiment.
Figure 2 illustrates a medication administration system, according to an example embodiment.
Figures 3A-3C illustrate another medication administration system, according to an example embodiment.
Figure 4 is a block diagram of a method for confirming one or more tubes of a medicament administration system, according to an example embodiment.

### Detailed Description

Example methods and systems are described herein. It should be understood that the words "example," "exemplary," and "illustrative" are used herein to mean "serving as an example, instance, or illustration." Any embodiment or feature described herein as being an "example," being "exemplary," or being "illustrative" is not necessarily to be construed as preferred or advantageous over other embodiments or features. The example embodiments described herein are not meant to be limiting. It will be readily understood that the aspects of the present disclosure, as generally described herein, and illustrated in the figures, can be arranged, substituted, combined, separated, and designed in a wide variety of different configurations, all of which are explicitly contemplated herein.

Furthermore, the particular arrangements shown in the Figures should not be viewed as limiting. It should be understood that other embodiments may include more or less of each element shown in a given Figure. Further, some of the illustrated elements may be combined or omitted. Yet further, an example embodiment may include elements that are not illustrated in the Figures.

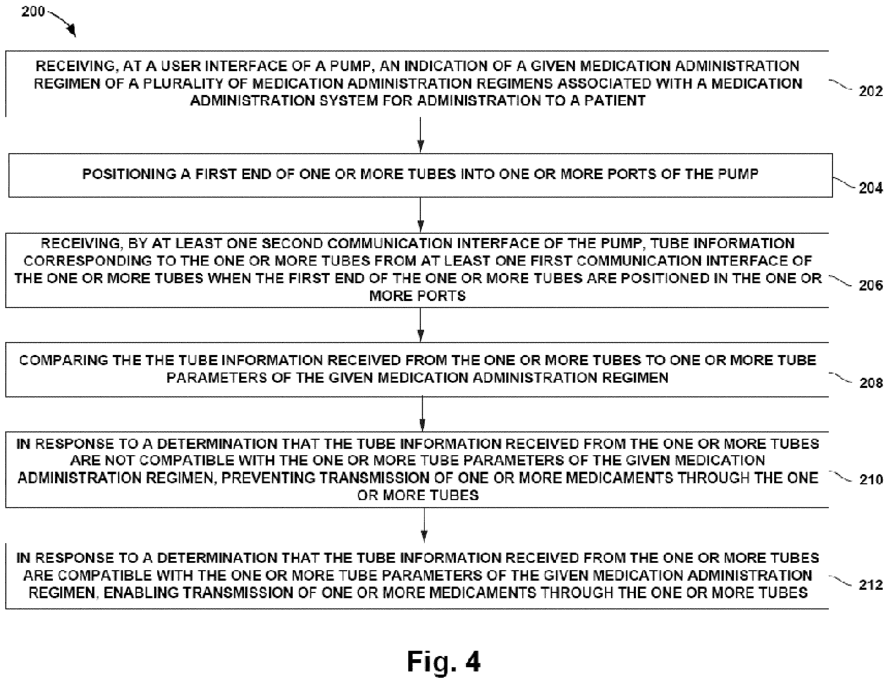

In Figure 4, referred to above, the blocks may represent operations and/or portions thereof and lines connecting the various blocks do not imply any particular order or dependency of the operations or portions thereof. It will be understood that not all dependencies among the various disclosed operations are necessarily represented. Figure 4 and the accompanying disclosure describing the operations of the method(s) set forth herein should not be interpreted as necessarily determining a sequence in which the operations are to be performed. Rather, although one illustrative order is indicated, it is to be understood that the sequence of the operations may be modified when appropriate. Accordingly, certain operations may be performed in a different order or simultaneously. Additionally, those skilled in the art will appreciate that not all operations described need be performed.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

Reference herein to "one embodiment" or "one example" means that one or more feature, structure, or characteristic described in connection with the example is included in at least one implementation. The phrases "one embodiment" or "one example" in various places in the specification may or may not be referring to the same example.

As used herein, apparatus, element and method "configured to" perform a specified function is indeed capable of performing the specified function without any alteration, rather than merely having potential to perform the specified function after further modification. In other words, the apparatus, element, and method "configured to" perform a specified function is specifically selected, created, implemented, utilized, programmed, and/or designed for the purpose of performing the specified function. As used herein, "configured to" refers to existing characteristics of an apparatus, element, and method which enable the apparatus, element, and method to perform the specified function without further modification. For purposes of this disclosure, an apparatus, element, and method described as being "configured to" perform a particular function can additionally or alternatively be described as being "adapted to" and/or as being "operative to" perform that function.

Further, in the following description, the wording medicament administration system will be used. In this context, medicament administration systems may include a number of devices capable of delivering certain doses of medicament to a user. The medicament administration systems may be of either disposable type or re-usable type and may be provided with medicament containers suitably arranged for specific drugs in specific forms.

Principles and embodiments of the present disclosure relate to an improved system for infusion set tubing attachment to a medication administration system. More particularly, the present disclosure relates to the facilitation and verification system to confirm one or more tubing sets are configured properly in their attachment to a medication administration system. As discussed in additional detail below, multiple tubing sets may or may not be housed in a single attachment configured by a manufacturer, pharmacist, or other healthcare professional. The tubing sets, either individually or within the housing, will encompass one or more communication interface(s) (e.g., short range communication technology) to communicate with the pump of the medication administration system. Examples of such communication interface(s) include but are not limited to radio frequency identification (RFID), near-field communication (NFC), Bluetooth, Bluetooth low energy (BLE), Ultra wide band (UWB), wireless fidelity (Wi-Fi), and infrared (IR). The tubing or housing could also utilize direct electronic and/or optical connection. Aspects of the tubing set communicated between components of the medication administration system may include, by way of example but not limitation, product identification parameters, tubing flow rate, tubing size, tubing volume for priming, and air or occlusion detection data.

The medication administration system may be equipped with such technology to receive this information and confirm its appropriateness for a given medication administration regimen. It may confirm information including but not limited to: that each tubing set is authentic, the correct tubing is attached to the correct port in the correct manner for the entire configuration, the correct number of tubing sets are attached, and the precise volume for priming each tubing set. If not appropriate, the system may give a warning or fail to proceed with administration. The precise priming volume will be delivered automatically, with or without user confirmation.

This information may be "programmed" or "tagged" onto the housing or individual tubing sets by the manufacturer, or by a healthcare professional such as a pharmacist during preparation. This could work as part of an electronic dispensing system for specialty pharmacies, where order information is stored. The information from the electronic dispensing system could be easily "zapped" or "scanned" onto the tubing sets/housing by the pharmacy during dispensing, by, for example, programming a near-field communication chip. Information on the tubing set could be similarly programmed and/or used in a manner to the approaches disclosed in, for example, EP3856293A1 or EP3639871, with a tubing set instead of a drug cartridge or autoinjector.

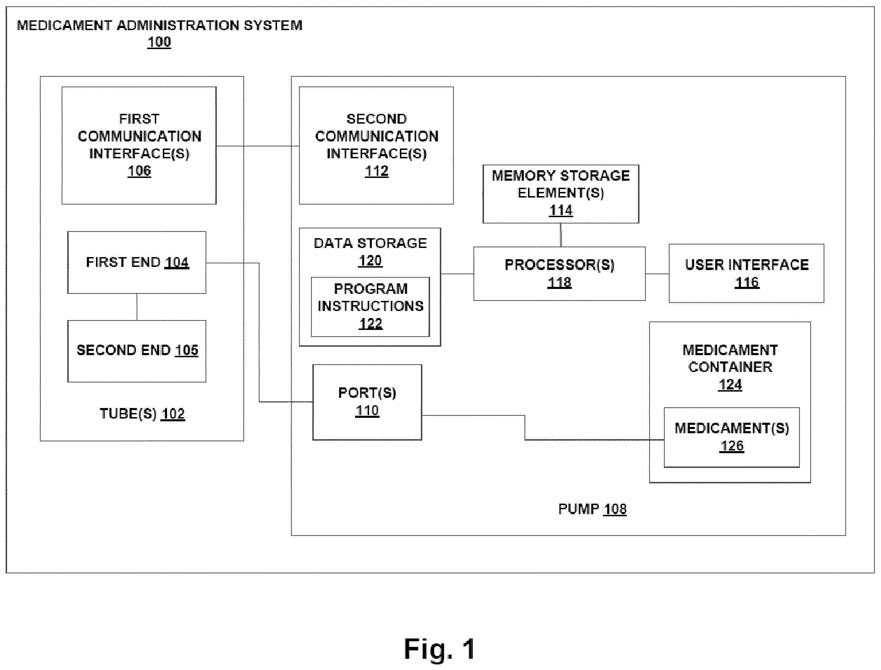

With reference to the figures, Figure 1 illustrates a simplified block diagram of a medicament administration system 100, according to an example embodiment. As shown in Figure 1, the medicament administration system 100 includes one or more tubes 102 each having a first end 104 and a second end 105 opposite the first end 104. The one or more tubes 102 further include at least one first communication interface 106 configured to transmit tube information corresponding to the one or more tubes 102. The tube information corresponding to the one or more tubes may comprise one or more of a product identification parameter, a tubing flow rate, a tubing size, a tubing volume for priming, and an air or occlusion detection data, as non-limiting examples. In one example, the tube information corresponding to the one or more tubes 102 is provided to the at least one first communication interface 106 during manufacturing of the one or more tubes 102. In another example, the tube information corresponding to the one or more tubes 102 is provided to the at least one first communication interface 106 by a healthcare professional after manufacturing of the one or more tubes 102. This could work as part of an electronic dispensing system for specialty pharmacies, where order information is stored. The information from the electronic dispensing system could be easily "zapped" or "scanned" onto the one or more tubes 102 by the pharmacy during dispensing.

The medicament administration system 100 further includes a pump 108. The pump 108 includes one or more ports 110 configured to receive the first end 104 of the one or more tubes 102. The pump 108 further includes at least one second communication interface 112 configured to receive the tube information from the at least one first communication interface 106 when the first end 104 of the one or more tubes 102 are positioned in the one or more ports 110. The at least one first communication interface 106 and the at least one second communication interface 112 may contain hardware to enable a communication link therebetween, such as processors, transmitters, receivers, antennas, etc., as discussed above. In one example, the communication link between the at least one first communication interface 106 and the at least one second communication interface 112 is a wired connection. In another example, the communication link between the at least one first communication interface 106 and the at least one second communication interface 112 is a wireless connection such as radio frequency identification (RFID), near-field communication (NFC), Bluetooth, Bluetooth low energy (BLE), Ultra wide band (UWB), wireless fidelity (Wi-Fi), and infrared (IR), as non-limiting examples.

The pump 108 further includes at least one memory storage element 114 including a plurality of medication administration regimens associated with the medication administration system. The at least one memory storage element 114 can include any type of memory now known or later developed including but not limited to volatile memory (such as RAM), non-volatile memory (such as ROM, flash memory, etc.) or any combination thereof. The pump 108 also includes a user interface 116 configured to receive an indication of a given medication administration regimen of the plurality of medication administration regimens for administration to a patient. The user interface 116 may be, for example, an optical see-through display, an optical see-around display, or a video see-through display, as non-limiting examples.

The pump 108 further includes at least one processor 118 and data storage 120 including program instructions 122 stored thereon that when executed by the at least one processor 118, cause the pump 108 to perform functions. Although various components of the medicament administration system 100 are shown as distributed components, it should be understood that any of such components may be physically integrated and/or distributed according to the desired configuration of the system. Depending on the desired configuration, the at least one processor 118 can be any type of processor including, but not limited to, a microprocessor, a microcontroller, a digital signal processor, or any combination thereof. In particular, the functions include (i) comparing the the tube information received from the one or more tubes 102 to one or more tube parameters of the given medication administration regimen, (ii) in response to a determination that the tube information received from the one or more tubes 102 are not compatible with the one or more tube parameters of the given medication administration regimen, preventing transmission of one or more medicaments through the one or more tubes 102, and (iii) in response to a determination that the tube information received from the one or more tubes 102 are compatible with the one or more tube parameters of the given medication administration regimen, enabling transmission of one or more medicaments through the one or more tubes 102.

The one or more tube parameters of the given medication administration regimen may comprise one or more of a tubing product, a tubing flow rate, a tubing size, a number of tubes, a positioning of tubes, and a tubing volume for priming, as non-limiting examples.

In one example, as shown in Figure 1, the pump 108 further includes a medicament container 124 including one or more medicaments 126 configured to be pumped through the one or more tubes 102 though the one or more ports 110.

In one example, the pump 108 is further configured to, in response to the determination that the tube information received from the one or more tubes 102 does not match the one or more tube parameters of the given medication administration regimen, provide for display a warning message on the user interface 116. Such a warning message may be a visual message and/or an audible message.

In another example, the pump 108 is further configured to, in response to the determination that the tube information received from the one or more tubes matches the one or more tube parameters of the given medication administration regimen, automatically prime each of the one or more tubes.

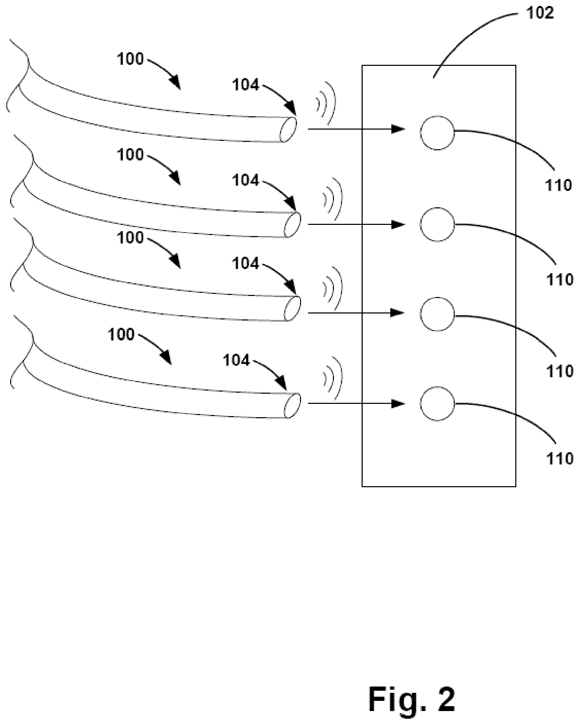

In one example, as shown in Figure 2, each of the one or more tubes 102 includes a first communication interface 106 to communicate with the pump 108. In one particular example, the first communication interface 106 of each of the one or more tubes 102 is positioned at the first end 104 of each of the one or more tubes 102. In one example, the one or more ports 110 may each include a second communication interface 112 configured to receive the tube information from the one or more tubes 102.

As shown in Figure 2, in use each of the one or more tubes 102 are individually attached to the corresponding one or more ports 110 by either the patient or healthcare professional. Information from each of the one or more tubes 102 is communicated to the pump 108 and verified. For example, the pump 108 may verify that each tube 102 is in the correct port 110 and meets the size, flow rate, and length requirements for the given medication administration regimen. The priming volume of each of the one or more tubes 102 may be communicated and the pump 108 can accurately prime each tube automatically, with or without user confirmation. If any of the information communicated by the one or more tubes 102 to the pump 108 is not compatible with the one or more tube parameters of the given medication administration regimen, the system may prevent administration of the medicament and/or provide a warning message as discussed above.

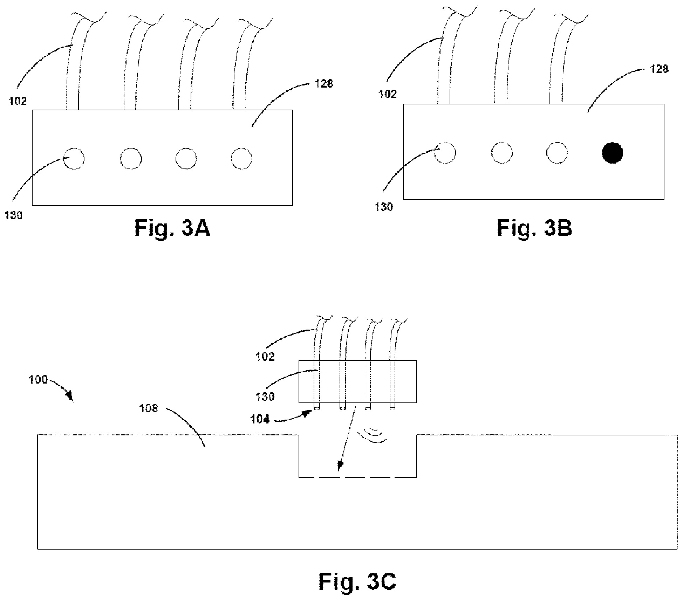

In another example, as shown in Figures 3A-3C, the one or more tubes 102 further comprise a housing 128 including one or more through holes 130, and the first end 104 of the one or more tubes 102 are positioned though the one or more through holes 130. In one such example, each of the one or more tubes 102 includes a first communication interface 106. In another example, the housing 128 includes a single first communication interface 106 that contains information relating to all of the tubes connected to the housing 128.

As shown in Figures 3A-3B, the one or more tubes 102 are configured into a housing 128 by either a healthcare professional or manufacturer. As described above, the first communication interface 106 may either be in each of the one or more tubes 102 or just in the housing 128. The housing 128 may be "programmed" or "tagged" by either the manufacturer or a healthcare professional with the tube information as discussed above. As shown in Figure 3C, the housing will be "plugged in" to the pump 108 by either the patient or healthcare professional. The first communication interface 106 will communicate the configuration of tubing for the housing 128, and ensure that the correct ports 110 are aligned. Additional information may also be communicated by the housing 128 and verified by the pump 108. Examples of this information include verification that each tube meets the size, flow rate, and length requirements for the patient and the medication. The priming volume of each of the one or more tubes 102 may be communicated and the pump 108 can accurately prime each tube automatically, with or without user confirmation. If any of the information communicated by the one or more tubes 102 to the pump 108 is not compatible with the one or more tube parameters of the given medication administration regimen, the system may prevent administration of the medicament and/or provide a warning message as discussed above.

As shown in Figure 3B, if less than the maximum number of ports 110 are being used by a particular patient, the housing 128 remains the same, and simply one less port is used when the housing 128 is being prepared. This allows for the same use steps for patients who change therapies or number of medications, simply "plug and play", hiding complexity for the patient.

Figure 4 is a block diagram of an example method for confirming one or more tubes of a medicament administration system. Method 200 shown in Figure 4 presents an embodiment of a method that could be used by the medicament administration system 100 as described in Figures 1-3C, as examples. Method 200 may include one or more operations, functions, or actions as illustrated by one or more of blocks 202-212. Although the blocks are illustrated in a sequential order, these blocks may also be performed in parallel, and/or in a different order than those described herein. Also, the various blocks may be combined into fewer blocks, divided into additional blocks, and/or removed based upon the desired implementation.

In addition, for the method 200 and other processes and methods disclosed herein, the block diagram shows functionality and operation of one possible implementation of present embodiments. In this regard, each block may represent a module, a segment, or a portion of program code, which includes one or more instructions executable by a processor or computing device for implementing specific logical functions or steps in the process. The program code may be stored on any type of computer readable medium, for example, such as a storage device including a disk or hard drive. The computer readable medium may include non-transitory computer readable medium, for example, such as computer-readable media that stores data for short periods of time like register memory, processor cache and Random Access Memory (RAM). The computer readable medium may also include non-transitory media, such as secondary or persistent long term storage, like read only memory (ROM), optical or magnetic disks, compact-disc read only memory (CD-ROM), for example. The computer readable media may also be any other volatile or non-volatile storage systems. The computer readable medium may be considered a computer readable storage medium, for example, or a tangible storage device.

Initially, at block 202, the method 200 includes receiving, at a user interface 116 of a pump 108, an indication of a given medication administration regimen of a plurality of medication administration regimens associated with a medicament administration system 100 for administration to a patient. At block 204, the method 200 includes positioning a first end 104 of one or more tubes 102 into one or more ports 110 of the pump 108. At block 206, the method 200 includes receiving, by at least one second communication interface 112 of the pump 108, tube information corresponding to the one or more tubes from at least one first communication interface 106 of the one or more tubes 102 when the first end 104 of the one or more tubes 102 are positioned in the one or more ports 110. At block 208, the method 200 includes comparing the the tube information received from the one or more tubes 102 to one or more tube parameters of the given medication administration regimen. At block 210, the method 200 includes, in response to a determination that the tube information received from the one or more tubes 102 are not compatible with the one or more tube parameters of the given medication administration regimen, preventing transmission of one or more medicaments through the one or more tubes 102. At block 212, the method 200 includes, in response to a determination that the tube information received from the one or more tubes 102 are compatible with the one or more tube parameters of the given medication administration regimen, enabling transmission of one or more medicaments through the one or more tubes 102.

In one example, the method 200 further includes, in response to the determination that the tube information received from the one or more tubes 102 does not match the one or more tube parameters of the given medication administration regimen, providing for display a warning message on the user interface 116. In another example, the method 200 further includes, in response to the determination that the tube information received from the one or more tubes 102 matches the one or more tube parameters of the given medication administration regimen, automatically priming each of the one or more tubes 102.

It will be appreciated that other arrangements are possible as well, including some arrangements that involve more or fewer steps than those described above, or steps in a different order than those described above.

While various aspects and embodiments have been disclosed herein, other aspects and embodiments will be apparent to those skilled in the art. All embodiments within and between different aspects of the devices and methods can be combined unless the context clearly dictates otherwise. The various aspects and embodiments disclosed herein are for purposes of illustration and are not intended to be limiting, with the true scope and spirit being indicated by the claims.

The delivery devices described herein can be used for the treatment and/or prophylaxis of one or more of many different types of disorders.

Exemplary disorders include, but are not limited to: rheumatoid arthritis, inflammatory bowel diseases (e.g. Crohn's disease and ulcerative colitis), hypercholesterolaemia and/or dyslipidemia, cardiovascular disease, diabetes (e.g. type 1 or 2 diabetes), psoriasis, psoriatic arthritis, spondyloarthritis, hidradenitis suppurativa, Sjögren's syndrome, migraine, cluster headache, multiple sclerosis, neuromyelitis optica spectrum disorder, anaemia, thalassemia, paroxysmal nocturnal hemoglobinuria, hemolytic anaemia, hereditary angioedema, systemic lupus erythematosus, lupus nephritis, myasthenia gravis, Behçet's disease, hemophagocytic lymphohistiocytosis, atopic dermatitis, retinal diseases (e.g., age-related macular degeneration, diabetic macular edema), uveitis, infectious diseases, bone diseases (e.g., osteoporosis, osteopenia), asthma, chronic obstructive pulmonary disease, thyroid eye disease, nasal polyps, transplant, acute hypoglycaemia, obesity, anaphylaxis, allergies, sickle cell disease, Alzheimer's disease, Parkinson's disease, dementia with Lewy bodies, systemic infusion reactions, immunoglobulin E (IgE)-mediated hypersensitivity reactions, cytokine release syndrome, immune deficiencies (e.g., primary immunodeficiency, chronic inflammatory demyelinating polyneuropathy), enzyme deficiencies (e.g., Pompe disease, Fabry disease, Gaucher disease), growth factor deficiencies, hormone deficiencies, coagulation disorders (e.g., hemophilia, von Willebrand disease, Factor V Leiden), and cancer.

Exemplary types of drugs that could be included in the delivery devices described herein include, but are not limited to, small molecules, hormones, cytokines, blood products, enzymes, vaccines, anticoagulants, immunosuppressants, antibodies, antibody-drug conjugates, neutralizing antibodies, reversal agents, radioligand therapies, radioisotopes and/or nuclear medicines, diagnostic agents, bispecific antibodies, proteins, fusion proteins, peptibodies, polypeptides, pegylated proteins, protein fragments, nucleotides, protein analogues, protein variants, protein precursors, protein derivatives, chimeric antigen receptor T cell therapies, cell or gene therapies, oncolytic viruses, or immunotherapies.

Exemplary drugs that could be included in the delivery devices described herein include, but are not limited to, immuno-oncology or bio-oncology medications such as immune checkpoints, cytokines, chemokines, clusters of differentiation, interleukins, integrins, growth factors, coagulation factors, enzymes, enzyme inhibitors, retinoids, steroids, signaling proteins, pro-apoptotic proteins, anti-apoptotic proteins, T-cell receptors, B-cell receptors, or costimulatory proteins.

Exemplary drugs that could be included in the delivery devices described herein include, but are not limited to, those exhibiting a proposed mechanism of action, such as human epidermal growth factor receptor 2 (HER-2) receptor modulators, interleukin (IL) modulators, interferon (IFN) modulators, complement modulators, glucagon-like peptide-1 (GLP-1) modulators, glucose-dependent insulinotropic polypeptide (GIP) modulators, cluster of differentiation 38 (CD38) modulators, cluster of differentiation 22 (CD22) modulators, C1 esterase modulators, bradykinin modulators, C-C chemokine receptor type 4 (CCR4) modulators, vascular endothelial growth factor (VEGF) modulators, B-cell activating factor (BAFF), P-selectin modulators, neonatal Fc receptor (FcRn) modulators, calcitonin gene-related peptide (CGRP) modulators, epidermal growth factor receptor (EGFR) modulators, cluster of differentiation 79B (CD79B) modulators, tumor-associated calcium signal transducer 2 (Trop-2) modulators, cluster of differentiation 52 (CD52) modulators, B-cell maturation antigen (BCMA) modulators, enzyme modulators, platelet-derived growth factor receptor A (PDGFRA) modulators, cluster of differentiation 319 (CD319 or SLAMF7) modulators, programmed cell death protein 1 and programmed death-ligand 1 (PD-1/PD-L1) inhibitors/modulators, B-lymphocyte antigen cluster of differentiation 19 (CD19) inhibitors, B-lymphocyte antigen cluster of differentiation 20 (CD20) modulators, cluster of differentiation 3 (CD3) modulators, cytotoxic T-lymphocyte-associated protein 4 (CTLA-4) inhibitors, T-cell immunoglobulin and mucin-domain containing-3 (TIM-3) modulators, T cell immunoreceptor with Ig and ITIM domains (TIGIT) modulators, V-domain Ig suppressor of T cell activation (VISTA) modulators, indoleamine 2,3-dioxygenase (IDO or INDO) modulators, poliovirus receptor-related immunoglobulin domain-containing protein (PVRIG) modulators, lymphocyte-activation gene 3 (LAG3; also known as cluster of differentiation 223 or CD223) antagonists, cluster of differentiation 276 (CD276 or B7-H3) antigen modulators, cluster of differentiation 47 (CD47) antagonists, cluster of differentiation 30 (CD30) modulators, cluster of differentiation 73 (CD73) modulators, cluster of differentiation 66 (CD66) modulators, cluster of differentiation w137 (CDw137) agonists, cluster of differentiation 158 (CD158) modulators, cluster of differentiation 27 (CD27) modulators, cluster of differentiation 58 (CD58) modulators, cluster of differentiation 80 (CD80) modulators, cluster of differentiation 33 (CD33) modulators, cluster of differentiation 159 (CD159 or NKG2) modulators, glucocorticoid-induced TNFR-related (GITR) protein modulators, Killer Ig-like receptor (KIR) modulators, growth arrest-specific protein 6 (GAS6)/AXL pathway modulators, A proliferation-inducing ligand (APRIL) receptor modulators, human leukocyte antigen (HLA) modulators, epidermal growth factor receptor (EGFR) modulators, B-lymphocyte cell adhesion molecule modulators, cluster of differentiation w123 (CDw123) modulators, Erbb2 tyrosine kinase receptor modulators, endoglin modulators, mucin modulators, mesothelin modulators, hepatitis A virus cellular receptor 2 (HAVCR2) antagonists, cancer-testis antigen (CTA) modulators, tumor necrosis factor receptor superfamily, member 4 (TNFRSF4 or OX40) modulators, adenosine receptor modulators, inducible T cell co-stimulator (ICOS) modulators, cluster of differentiation 40 (CD40) modulators, tumor-infiltrating lymphocytes (TIL) therapies, or T-cell receptor (TCR) therapies.

Exemplary drugs that could be included in the delivery devices described herein include, but are not limited to: etanercept, abatacept, adalimumab, evolocumab, exenatide, secukinumab, erenumab, galcanezumab, fremanezumab-vfrm, alirocumab, methotrexate (amethopterin), tocilizumab, interferon beta-1a, interferon beta-1b, peginterferon beta-1a, sumatriptan, darbepoetin alfa, belimumab, sarilumab, semaglutide, dupilumab, reslizumab, omalizumab, glucagon, epinephrine, naloxone, insulin, amylin, vedolizumab, eculizumab, ravulizumab, crizanlizumab-tmca, certolizumab pegol, satralizumab, denosumab, romosozumab, benralizumab, emicizumab, tildrakizumab, ocrelizumab, ofatumumab, natalizumab, mepolizumab, risankizumab-rzaa, ixekizumab, and immune globulins.

Exemplary drugs that could be included in the delivery devices described herein may also include, but are not limited to, oncology treatments such as ipilimumab, nivolumab, pembrolizumab, atezolizumab, durvalumab, avelumab, cemiplimab, rituximab, trastuzumab, ado-trastuzumab emtansine, fam-trastuzumab deruxtecan-nxki, pertuzumab, transtuzumab-pertuzumab, alemtuzumab, belantamab mafodotin-blmf, bevacizumab, blinatumomab, brentuximab vedotin, cetuximab, daratumumab, elotuzumab, gemtuzumab ozogamicin, 90-Yttrium-ibritumomab tiuxetan, isatuximab, mogamulizumab, moxetumomab pasudotox, obinutuzumab, ofatumumab, olaratumab, panitumumab, polatuzumab vedotin, ramucirumab, sacituzumab govitecan, tafasitamab, or margetuximab.

Exemplary drugs that could be included in the delivery devices described herein include "generic" or biosimilar equivalents of any of the foregoing, and the foregoing molecular names should not be construed as limiting to the "innovator" or "branded" version of each, as in the non-limiting example of innovator medicament adalimumab and biosimilars such as adalimumab-afzb, adalimumab-atto, adalimumab-adbm, and adalimumab-adaz.

Exemplary drugs that could be included in the delivery devices described herein also include, but are not limited to, those used for adjuvant or neoadjuvant chemotherapy, such as an alkylating agent, plant alkaloid, antitumor antibiotic, antimetabolite, or topoisomerase inhibitor, enzyme, retinoid, or corticosteroid. Exemplary chemotherapy drugs include, by way of example but not limitation, 5-fluorouracil, cisplatin, carboplatin, oxaliplatin, doxorubicin, daunorubicin, idarubicin, epirubicin, paclitaxel, docetaxel, cyclophosphamide, ifosfamide, azacitidine, decitabine, bendamustine, bleomycin, bortezomib, busulfan, cabazitaxel, carmustine, cladribine, cytarabine, dacarbazine, etoposide, fludarabine, gemcitabine, irinotecan, leucovorin, melphalan, methotrexate, pemetrexed, mitomycin, mitoxantrone, temsirolimus, topotecan, valrubicin, vincristine, vinblastine, or vinorelbine.

Exemplary drugs that could be included in the delivery devices described herein also include, but are not limited to, analgesics (e.g., acetaminophen), antipyretics, corticosteroids (e.g. hydrocortisone, dexamethasone, or methylprednisolone), antihistamines (e.g., diphenhydramine or famotidine), antiemetics (e.g., ondansetron), antibiotics, antiseptics, anticoagulants, fibrinolytics (e.g., recombinant tissue plasminogen activator [r-TPA]), antithrombolytics, or diluents such as sterile water for injection (SWFI), 0.9% Normal Saline, 0.45% normal saline, 5% dextrose in water, 5% dextrose in 0.45% normal saline, Lactated Ringer's solution, Heparin Lock Flush solution, 100 U/mL Heparin Lock Flush Solution, or 5000 U/mL Heparin Lock Flush Solution.

Pharmaceutical formulations including, but not limited to, any drug described herein are also contemplated for use in the delivery devices described herein, for example pharmaceutical formulations comprising a drug as listed herein (or a pharmaceutically acceptable salt of the drug) and a pharmaceutically acceptable carrier. Such formulations may include one or more other active ingredients (e.g., as a combination of one or more active drugs), or may be the only active ingredient present, and may also include separately administered or co-formulated dispersion enhancers (e.g. an animal-derived, human-derived, or recombinant hyaluronidase enzyme), concentration modifiers or enhancers, stabilizers, buffers, or other excipients.

Exemplary drugs that could be included in the delivery devices described herein include, but are not limited to, a multi-medication treatment regimen such as AC, Dose-Dense AC, TCH, GT, EC, TAC, TC, TCHP, CMF, FOLFOX, mFOLFOX6, mFOLFOX7, FOLFCIS, CapeOx, FLOT, DCF, FOLFIRI, FOLFIRINOX, FOLFOXIRI, IROX, CHOP, R-CHOP, RCHOP-21, Mini-CHOP, Maxi-CHOP, VR-CAP, Dose-Dense CHOP, EPOCH, Dose-Adjusted EPOCH, R-EPOCH, CODOX-M, IVAC, HyperCVAD, R-HyperCVAD, SC-EPOCH-RR, DHAP, ESHAP, GDP, ICE, MINE, CEPP, CDOP, GemOx, CEOP, CEPP, CHOEP, CHP, GCVP, DHAX, CALGB 8811, HIDAC, MOpAD, 7 + 3, 5 +2, 7 + 4, MEC, CVP, RBAC500, DHA-Cis, DHA-Ca, DHA-Ox, RCVP, RCEPP, RCEOP, CMV, DDMVAC, GemFLP, ITP, VIDE, VDC, VAI, VDC-IE, MAP, PCV, FCR, FR, PCR, HDMP, OFAR, EMA/CO, EMA/EP, EP/EMA, TP/TE, BEP, TIP, VIP, TPEx, ABVD, BEACOPP, AVD, Mini-BEAM, IGEV, C-MOPP, GCD, GEMOX, CAV, DT-PACE, VTD-PACE, DCEP, ATG, VAC, VeIP, OFF, GTX, CAV, AD, MAID, AIM, VAC-IE, ADOC, or PE.

## Claims

1. A medication administration system (100) comprising:
one or more tubes (102) having a first end (104) and a second end (105) opposite the first end, the one or more tubes including:
at least one first communication interface (106) configured to transmit tube information corresponding to the one or more tubes; and
a pump (108) including:
one or more ports (110) configured to receive the first end of the one or more tubes;
at least one second communication interface (112) configured to receive the tube information from the at least one first communication interface when the first end of the one or more tubes are positioned in the one or more ports;
at least one memory storage element (114) including a plurality of medication administration regimens associated with the medication administration system;
a user interface (116) configured to receive an indication of a given medication administration regimen of the plurality of medication administration regimens for administration to a patient;
at least one processor (118); and
data storage (120) including program instructions (122) stored thereon that when executed by the at least one processor, cause the pump to:
compare the the tube information received from the one or more tubes to one or more tube parameters of the given medication administration regimen;
in response to a determination that the tube information received from the one or more tubes are not compatible with the one or more tube parameters of the given medication administration regimen, prevent transmission of one or more medicaments through the one or more tubes; and
in response to a determination that the tube information received from the one or more tubes are compatible with the one or more tube parameters of the given medication administration regimen, enable transmission of one or more medicaments through the one or more tubes.

2. The medication administration system of claim 1, wherein each of the one or more tubes includes a first communication interface.

3. The medication administration system of claim 2, wherein the first communication interface of each of the one or more tubes is positioned at the first end of each of the one or more tubes.

4. The medication administration system of any one of claims 1-3, wherein the one or more tubes further comprise a housing including one or more through holes, and wherein the first end of the one or more tubes are positioned though the one or more through holes.

5. The medication administration system of claim 4, wherein each of the one or more tubes includes a first communication interface.

6. The medication administration system of claim 4, wherein the housing includes a single first communication interface.

7. The medication administration system of any one of claims 1-6, wherein the pump is further configured to:
in response to the determination that the tube information received from the one or more tubes does not match the one or more tube parameters of the given medication administration regimen, provide for display a warning message on the user interface.

8. The medication administration system of any one of claims 1-7, wherein the pump is further configured to:
in response to the determination that the tube information received from the one or more tubes matches the one or more tube parameters of the given medication administration regimen, automatically prime each of the one or more tubes.

9. The medication administration system of any one of claims 1-8, wherein the tube information corresponding to the one or more tubes comprises one or more of a product identification parameter, a tubing flow rate, a tubing size, a tubing volume for priming, and an air or occlusion detection data.

10. The medication administration system of any one of claims 1-9, wherein the one or more tube parameters of the given medication administration regimen comprises one or more of a tubing product, a tubing flow rate, a tubing size, a number of tubes, a positioning of tubes, and a tubing volume for priming.

11. The medication administration system of any one of claims 1-10, wherein the tube information corresponding to the one or more tubes is provided to the at least one first communication interface during manufacturing of the one or more tubes.

12. The medication administration system of any one of claims 1-10, wherein the tube information corresponding to the one or more tubes is provided to the at least one first communication interface by a healthcare professional after manufacturing of the one or more tubes.

13. A method for confirming one or more tubes of a medicament administration system, the method comprising:
receiving, at a user interface of a pump, an indication of a given medication administration regimen of a plurality of medication administration regimens associated with a medication administration system for administration to a patient;
positioning a first end of one or more tubes into one or more ports of the pump;
receiving, by at least one second communication interface of the pump, tube information corresponding to the one or more tubes from at least one first communication interface of the one or more tubes when the first end of the one or more tubes are positioned in the one or more ports;
comparing the the tube information received from the one or more tubes to one or more tube parameters of the given medication administration regimen;
in response to a determination that the tube information received from the one or more tubes are not compatible with the one or more tube parameters of the given medication administration regimen, preventing transmission of one or more medicaments through the one or more tubes; and
in response to a determination that the tube information received from the one or more tubes are compatible with the one or more tube parameters of the given medication administration regimen, enabling transmission of one or more medicaments through the one or more tubes.

14. The method of claim 13, further comprising:
in response to the determination that the tube information received from the one or more tubes does not match the one or more tube parameters of the given medication administration regimen, providing for display a warning message on the user interface.

15. The method of any one of claims 13-14, further comprising:
in response to the determination that the tube information received from the one or more tubes matches the one or more tube parameters of the given medication administration regimen, automatically priming each of the one or more tubes.

## Patentansprüche

1. Medikamentenverabreichungssystem (100), umfassend:
einen oder mehrere Schläuche (102), die ein erstes Ende (104) und ein zweites Ende (105) gegenüber dem ersten Ende aufweisen, wobei der eine oder die mehreren Schläuche einschließen:
mindestens eine erste Kommunikationsschnittstelle (106), die konfiguriert ist, um Schlauchinformationen zu übertragen, die dem einen oder den mehreren Schläuchen entsprechen; und
eine Pumpe (108) einschließend:
einen oder mehrere Anschlüsse (110), die konfiguriert sind, um das erste Ende des einen oder der mehreren Schläuche zu empfangen;
mindestens eine zweite Kommunikationsschnittstelle (112), die konfiguriert ist, um die Schlauchinformationen von der mindestens einen ersten Kommunikationsschnittstelle zu empfangen, wenn das erste Ende des einen oder der mehreren Schläuche in dem einen oder den mehreren Anschlüssen positioniert ist;
mindestens ein Speicherelement (114) einschließlich einer Vielzahl von Medikamentenverabreichungsschemata, die mit dem Medikamentenverabreichungssystem verknüpft sind;
eine Benutzerschnittstelle (116), die konfiguriert ist, um eine Anzeige eines gegebenen Medikamentenverabreichungsschemas der Vielzahl von Medikamentenverabreichungsschemata zur Verabreichung an einen Patienten zu empfangen;
mindestens einen Prozessor (118); und
Datenspeicher (120) einschließlich darauf gespeicherter Programmanweisungen (122), die, wenn sie durch den mindestens einen Prozessor ausgeführt werden, die Pumpe veranlassen zum:
Vergleichen der von dem einen oder den mehreren Schläuchen empfangenen Schlauchinformationen mit einem oder mehreren Schlauchparametern des gegebenen Medikamentenverabreichungsschemas;
als Reaktion auf eine Bestimmung, dass die von dem einen oder den mehreren Schläuchen empfangenen Schlauchinformationen nicht mit dem einen oder den mehreren Schlauchparametern des gegebenen Medikamentenverabreichungsschemas kompatibel sind, Verhindern der Übertragung von einem oder mehreren Medikamenten durch den einen oder die mehreren Schläuche; und
als Reaktion auf eine Bestimmung, dass die von dem einen oder den mehreren Schläuchen empfangenen Schlauchinformationen mit dem einen oder den mehreren Schlauchparametern des gegebenen Medikamentenverabreichungsschemas kompatibel sind, Ermöglichen der Übertragung von einem oder mehreren Medikamenten durch den einen oder die mehreren Schläuche.

2. Medikamentenverabreichungssystem nach Anspruch 1, wobei jeder der ein oder mehreren Schläuche eine erste Kommunikationsschnittstelle einschließt.

3. Medikamentenverabreichungssystem nach Anspruch 2, wobei die erste Kommunikationsschnittstelle jedes der einen oder mehreren Schläuche am ersten Ende jedes der einen oder mehreren Schläuche positioniert ist.

4. Medikamentenverabreichungssystem nach einem der Ansprüche 1 bis 3, wobei der eine oder die mehreren Schläuche ferner ein Gehäuse umfassen, das ein oder mehrere Durchgangslöcher einschließt, und wobei das erste Ende des einen oder der mehreren Schläuche durch das eine oder die mehreren Durchgangslöcher positioniert ist.

5. Medikamentenverabreichungssystem nach Anspruch 4, wobei jeder der ein oder mehreren Schläuche eine erste Kommunikationsschnittstelle einschließt.

6. Medikamentenverabreichungssystem nach Anspruch 4, wobei das Gehäuse eine einzelne erste Kommunikationsschnittstelle einschließt.

7. Medikamentenverabreichungssystem nach einem der Ansprüche 1 bis 6, wobei die Pumpe ferner konfiguriert ist zum:
als Reaktion auf die Bestimmung, dass die von dem einen oder den mehreren Schläuchen empfangenen Schlauchinformationen nicht mit dem einen oder den mehreren Schlauchparametern des gegebenen Medikamentenverabreichungsschemas übereinstimmen, Bereitstellen der Anzeige einer Warnmeldung auf der Benutzerschnittstelle.

8. Medikamentenverabreichungssystem nach einem der Ansprüche 1 bis 7, wobei die Pumpe ferner konfiguriert ist zum:
als Reaktion auf die Bestimmung, dass die von dem einen oder den mehreren Schläuchen empfangenen Schlauchinformationen mit dem einen oder den mehreren Schlauchparametern des gegebenen Medikamentenverabreichungsschemas übereinstimmen, automatisches Primen jedes der einen oder der mehreren Schläuche.

9. Medikamentenverabreichungssystem nach einem der Ansprüche 1 bis 8, wobei die Schlauchinformationen, die dem einen oder den mehreren Schläuchen entsprechen, einen oder mehrere von einem Produktidentifikationsparameter, einer Schlauchdurchflussrate, einer Schlauchgröße, einem Schlauchvolumen zum Primen und Daten zur Erkennung von Luft oder Verstopfung umfassen.

10. Medikamentenverabreichungssystem nach einem der Ansprüche 1 bis 9, wobei der eine oder die mehreren Schlauchparameter des gegebenen Medikamentenverabreichungsschemas einen oder mehrere von einem Schlauchprodukt, einer Schlauchdurchflussrate, einer Schlauchgröße, einer Anzahl von Schläuchen, einer Positionierung von Schläuchen und einem Schlauchvolumen zum Primen umfassen.

11. Medikamentenverabreichungssystem nach einem der Ansprüche 1 bis 10, wobei die Schlauchinformationen, die dem einen oder den mehreren Schläuchen entsprechen, der mindestens einen ersten Kommunikationsschnittstelle während der Herstellung des einen oder der mehreren Schläuche bereitgestellt werden.

12. Medikamentenverabreichungssystem nach einem der Ansprüche 1 bis 10, wobei die Schlauchinformationen, die dem einen oder den mehreren Schläuchen entsprechen, der mindestens einen ersten Kommunikationsschnittstelle von einer Gesundheitsfachkraft während der Herstellung des einen oder der mehreren Schläuche bereitgestellt werden.

13. Verfahren zum Bestätigen eines oder mehrerer Schläuche eines Medikamentenverabreichungssystems, das Verfahren umfassend:
Empfangen, an einer Benutzerschnittstelle einer Pumpe, einer Anzeige eines gegebenen Medikamentenverabreichungsschemas aus einer Vielzahl von Medikamentenverabreichungsschemata, die mit einem Medikamentenverabreichungssystem verknüpft sind, zur Verabreichung an einen Patienten;
Positionieren eines ersten Endes von einem oder mehreren Schläuchen in einem oder mehreren Anschlüssen der Pumpe;
Empfangen, durch mindestens eine zweite Kommunikationsschnittstelle der Pumpe, von Schlauchinformationen, die dem einen oder den mehreren Schläuchen entsprechen, von mindestens einer ersten Kommunikationsschnittstelle des einen oder der mehreren Schläuche, wenn das erste Ende des einen oder der mehreren Schläuche in dem einen oder den mehreren Anschlüssen positioniert ist;
Vergleichen der von dem einen oder den mehreren Schläuchen empfangenen Schlauchinformationen mit einem oder mehreren Schlauchparametern des gegebenen Medikamentenverabreichungsschemas;
als Reaktion auf eine Bestimmung, dass die von dem einen oder den mehreren Schläuchen empfangenen Schlauchinformationen nicht mit dem einen oder den mehreren Schlauchparametern des gegebenen Medikamentenverabreichungsschemas kompatibel sind, Verhindern der Übertragung von einem oder mehreren Medikamenten durch den einen oder die mehreren Schläuche; und
als Reaktion auf eine Bestimmung, dass die von dem einen oder den mehreren Schläuchen empfangenen Schlauchinformationen mit dem einen oder den mehreren Schlauchparametern des gegebenen Medikamentenverabreichungsschemas kompatibel sind, Ermöglichen der Übertragung von einem oder mehreren Medikamenten durch den einen oder die mehreren Schläuche.

14. Verfahren nach Anspruch 13, ferner umfassend:
als Reaktion auf die Bestimmung, dass die von dem einen oder den mehreren Schläuchen empfangenen Schlauchinformationen nicht mit dem einen oder den mehreren Schlauchparametern des gegebenen Medikamentenverabreichungsschemas übereinstimmen, Bereitstellen der Anzeige einer Warnmeldung auf der Benutzerschnittstelle.

15. Verfahren nach einem der Ansprüche 13 bis 14, ferner umfassend:
als Reaktion auf die Bestimmung, dass die von dem einen oder den mehreren Schläuchen empfangenen Schlauchinformationen mit dem einen oder den mehreren Schlauchparametern des gegebenen Medikamentenverabreichungsschemas übereinstimmen, automatisches Primen jedes der einen oder der mehreren Schläuche.

## Revendications

1. Système d'administration de médication (100) comprenant :
un ou plusieurs tubes (102) ayant une première extrémité (104) et une seconde extrémité (105) opposée à la première extrémité, le ou les tubes comportant :
au moins une première interface de communication (106) configurée pour transmettre des informations de tube correspondant au ou aux tubes ; et
une pompe (108) comportant :
un ou plusieurs orifices (110) conçus pour recevoir la première extrémité du ou des tubes ;
au moins une seconde interface de communication (112) configurée pour recevoir les informations de tube en provenance de l'au moins une première interface de communication lorsque la première extrémité du ou des tubes est positionnée dans le ou les orifices ;
au moins un élément de stockage de mémoire (114) comportant une pluralité de schémas d'administration de médication associés au système d'administration de médication ;
une interface utilisateur (116) configurée pour recevoir une indication d'un schéma d'administration de médication donné de la pluralité de schémas d'administration de médication pour administration à un patient ;
au moins un processeur (118) ; et
un stockage de données (120) comportant des instructions de programme (122) stockées sur celui-ci qui lorsqu'elles sont exécutées par l'au moins un processeur, amènent la pompe à :
comparer les informations de tube reçues en provenance du ou des tubes à un ou plusieurs paramètres de tube du schéma d'administration de médication donné ;
en réponse à une détermination que les informations de tube reçues en provenance du ou des tubes ne sont pas compatibles avec le ou les paramètres de tube du schéma d'administration de médication donné, empêcher la transmission d'un ou plusieurs médicaments à travers le ou les tubes ; et
en réponse à une détermination que les informations de tube reçues en provenance du ou des tubes sont compatibles avec le ou les paramètres de tube du schéma d'administration de médication donné, permettre la transmission d'un ou plusieurs médicaments à travers le ou les tubes.

2. Système d'administration de médication selon la revendication 1, dans lequel chaque tube ou chacun des tubes comporte une première interface de communication.

3. Système d'administration de médication selon la revendication 2, dans lequel la première interface de communication de chaque tube ou chacun des tubes est positionnée au niveau de la première extrémité de chaque tube ou chacun des tubes.

4. Système d'administration de médication selon l'une quelconque des revendications 1 à 3, dans lequel le ou les tubes comprennent en outre un logement comportant un ou plusieurs trous traversants, et dans lequel la première extrémité du ou des tubes est positionnée à travers le ou les trous traversants.

5. Système d'administration de médication selon la revendication 4, dans lequel chaque tube ou chacun des tubes comporte une première interface de communication.

6. Système d'administration de médication selon la revendication 4, dans lequel le logement comporte une seule première interface de communication.

7. Système d'administration de médication selon l'une quelconque des revendications 1 à 6, dans lequel la pompe est configurée en outre pour :
en réponse à la détermination que les informations de tube reçues en provenance du ou des tubes ne concordent pas avec le ou les paramètres de tube du schéma d'administration de médication donné, fournir pour affichage un message d'avertissement sur l'interface utilisateur.

8. Système d'administration de médication selon l'une quelconque des revendications 1 à 7, dans lequel la pompe est configurée en outre pour :
en réponse à la détermination que les informations de tube reçues en provenance du ou des tubes concordent avec le ou les paramètres de tube du schéma d'administration de médication donné, amorcer automatiquement chaque tube ou chacun des tubes.

9. Système d'administration de médication selon l'une quelconque des revendications 1 à 8, dans lequel les informations de tube correspondant au ou aux tubes comprennent un ou plusieurs parmi un paramètre d'identification de produit, un débit d'écoulement de tubulure, une taille de tubulure, un volume de tubulure pour amorçage et des données de détection d'air ou d'occlusion.

10. Système d'administration de médication selon l'une quelconque des revendications 1 à 9, dans lequel le ou les paramètres de tube du schéma d'administration de médication donné comprennent un ou plusieurs parmi un produit de tubulure, un débit d'écoulement de tubulure, une taille de tubulure, un nombre de tubes, un positionnement des tubes, et un volume de tubulure pour amorçage.

11. Système d'administration de médication selon l'une quelconque des revendications 1 à 10, dans lequel les informations de tube correspondant au ou aux tubes sont fournies à l'au moins une première interface de communication pendant la fabrication du ou des tubes.

12. Système d'administration de médication selon l'une quelconque des revendications 1 à 10, dans lequel les informations de tube correspondant au ou aux tubes sont fournies à l'au moins une première interface de communication par un professionnel de la santé après la fabrication du ou des tubes.

13. Procédé permettant de confirmer un ou plusieurs tubes d'un système d'administration de médicament, le procédé comprenant :
la réception, au niveau d'une interface utilisateur d'une pompe, d'une indication d'un schéma d'administration de médication donné d'une pluralité de schémas d'administration de médication associés à un système d'administration de médication pour administration à un patient ;
le positionnement d'une première extrémité d'un ou plusieurs tubes dans un ou plusieurs orifices de la pompe ;
la réception, par au moins une seconde interface de communication de la pompe, d'informations de tube correspondant au ou aux tubes en provenance d'au moins une première interface de communication du ou des tubes lorsque la première extrémité du ou des tubes est positionnée dans le ou les orifices ;
la comparaison des informations de tube reçues en provenance du ou des tubes à un ou plusieurs paramètres de tube du schéma d'administration de médication donné ;
en réponse à une détermination que les informations de tube reçues en provenance du ou des tubes ne sont pas compatibles avec le ou les paramètres de tube du schéma d'administration de médication donné, le fait d'empêcher la transmission d'un ou plusieurs médicaments à travers le ou les tubes ; et
en réponse à une détermination que les informations de tube reçues en provenance du ou des tubes sont compatibles avec le ou les paramètres de tube du schéma d'administration de médication donné, le fait de permettre la transmission d'un ou plusieurs médicaments à travers le ou les tubes.

14. Procédé selon la revendication 13, comprenant en outre :
en réponse à la détermination que les informations de tube reçues en provenance du ou des tubes ne concordent pas avec le ou les paramètres de tube du schéma d'administration de médication donné, la fourniture pour affichage d'un message d'avertissement sur l'interface utilisateur.

15. Procédé selon l'une quelconque des revendications 13 à 14, comprenant en outre :
en réponse à la détermination que les informations de tube reçues en provenance du ou des tubes concordent avec le ou les paramètres de tube du schéma d'administration de médication donné, l'amorçage automatique de chaque tube ou chacun des tubes.
